# EUROPÄISCHE PATENTSCHRIFT

(11) **EP 0 924 513 B1**
(45) Veröffentlichungstag und Bekanntmachung des Hinweises auf die Patenterteilung: **25.11.2009**
(21) Anmeldenummer: 98123333.1
(22) Anmeldetag: 08.12.1998
(51) Int. Cl.: G01N 27/22

(54) **Verfahren und Vorrichtung zur Ermittlung von Anteilen fester Stoffe in einem Prüfgut**
Method and device for measuring the proportions of solid materials in a sample
Méthode et appareil pour la mesure de la proportion des solides dans un échantillon

(30) Priorität: 18.12.1997 CH 291897
(43) Veröffentlichungstag der Anmeldung: 23.06.1999
(73) Patentinhaber: Uster Technologies AG, 8610 Uster (CH)
(72) Erfinder: Joss, Rolf, 8810 Horgen (CH); Geiter, Paul, 8807 Freienbach (CH)
(74) Vertreter: Pliska, Pavel

(56) Entgegenhaltungen:
- EP-A- 0 390 093
- EP-A1- 0 207 377
- US-A- 4 240 027
- US-A- 4 881 025
- US-A- 5 594 163
- US-A- 5 604 441

## Beschreibung

Die Erfindung betrifft ein Verfahren und eine Vorrichtung zur Ermittlung von Anteilen fester Stoffe in einem Prüfgut.

Ein Beispiel für die Ermittlung von Anteilen fester Stoffe in einem Prüfgut ist die Erkennung von Fremdstoffen und Fremdfasern in einem teilen Gebilde. Dabei geschieht die Erkennung üblicherweise mit optischen Mitteln. Beispielsweise sollen Fremdstoffe und Fremdfasern an ihren Reflexionseigenschaffen erkannt werden, die in der überwiegenden Zahl bekannter Fälle von den Reflexionseigenschaften des reinen teilen Gebildes abweichen. Deshalb wird das textile Gebilde mit Licht beaufschlagt. Dabei wird das, durch das Prüfgut absorbierte Licht und/oder das reflektierte Licht erfasst. Momentane oder lokale Abweichungen der Menge des empfangenen Lichts geben einen Hinweis auf den gesuchten Anteil erwünschter und unerwünschter Stoffe.

Ein Nachteil solcher bekannter Verfahren und Vorrichtungen besteht darin, dass farblose, durchsichtige oder durchscheinende Stoffe wie beispielsweise Polypropylen-Folien, oder Stoffe mit ähnlicher Farbe, wie Seile oder Schnüre, die bei der Verpackung von textilen Rohstoffen wie Baumwolle usw. verwendet werden, nicht erkannt werden. Das führt dazu, dass Teile solcher Polypropylen-Folien, Seilen und Schnüren mit dem Rohstoff weiterverarbeitet werden, so dass diese später beispielsweise in einem Garn eingesponnen sind.

Nun ist es auch möglich, solche feste Stoffe wie Polypropylen-Teile, in einem Garn versponnen zu erkennen. Dabei geht man davon aus, dass diese Stoffe oder Polypropylen-Teile die Struktur des Garns verändern und beispielsweise insbesondere die Haarigkeit in einem Abschnitt des Garns verändern. Dies erkennt man bei einer Messung des Durchmessers, beispielsweise dadurch, dass sich die Masse des Garns verändert oder dass sich die Haarigkeit verändert, weil dort Polypropylen-Teile statt Haare vom Garn abstehen. Somit hat man in diesem Falle eben Fremdteile durch eine Messung des Durchmessers, der Masse oder der Haarigkeit zu erfassen versucht.

Ein Nachteil dieser Erfassung von Anteilen fester Stoffe oder von Fremdstoffen über eine Messung des Durchmessers oder der Haarigkeit ist darin zu sehen, dass viele Fremdstoffe nicht erfasst werden. Dies hauptsächlich dann, wenn sie nicht an der Oberfläche des Prüfguts auftreten. Das führt dazu, dass ungünstige Anteile fester Stoffe und Fremdstoffe zu einem Endprodukt führen, das geschwächt ist oder das Fehler aufweist. Es kann aber auch sein, dass ein solches Produkt in einer nachfolgenden Verarbeitung Schwierigkeiten auslöst und es nicht mehr gelingt ein einwandfreies Endprodukt herzustellen.

Aus der US 4,881,025 ist eine Vorrichtung mit einem Kondensator bekannt, in dem verpackte Güter untersucht werden können. Dabei werden verschiedene elektrische Grössen erfasst und mit entsprechenden vorgespeicherten Werten für bekannte Güter verglichen. Damit können die Güter identifiziert werden. Diese Vorrichtung eignet sich somit nicht für die differenzierte Erkennung von Anteilen fremder Stoffe in einem Prüfgut wie Garn, das langgestreckt ist und in dem Kondensator in seiner Längsrichtung bewegt wird. Diese Vorrichtung eignet sich nur zur Identifikation homogener Stoffe.

Aus der EP 207 377 ist ein Messaufbau für dielektrische Spektroskopie bekannt, mit dem elektrische Kennwerte wie Dielektrizitätszahl, Permeabilitätszahl und Verlustfaktor von Werkstoffen bestimmt werden können. Besonders berücksichtigt wird hier die Erfassung der Temperaturabhängigkeit und der Frequenzabhängigkeit solcher Kennwerte. Mit diesem Messaufbau soll insbesondere die Beurteilung und Bewertung von Werkstoffen verbessert werden. Für die Erfassung von Fremdstoffanteilen in einem längsbewegten textilen Prüfgut ist dieser Messaufbau aber nicht geeignet, denn für eine quantitative Analyse der Werkstoffe ist dieser Messaufbau nicht vorgesehen.

Aus der US 5,604,441 ist ein Gerät für die kontinuierliche Analyse von Schmierstoffen in einem Schmiersystem für einen Dieselmotor bekannt. Mit diesem Gerät soll der Zersetzungsgrad des Schmieröls bestimmt werden können. Dies geschieht in einem Magnetfeld, das ferromagnetische Partikel, die durch die Abnützung des Motors in das Schmieröl gelangen, einem Sensor zuführt. Dieses Gerät sondert ferromagnetische Partikel von anderen Partikeln im Öl ab, gibt aber keine Angabe über den Anteil fremder Stoffe im Öl an. Es kann aber nicht für die Bestimmung von Anteilen fremder Stoffe in textilen Garnen verwendet werden, denn die Fremdstoffe sind im Garn ortsfest gebunden.

US 5,594,163 offenbart eine Vorrichtung zur Bestimmung des Zuzammensetzung von Benzin gemischen durch Analyse der Permittivität und elektrischer leitfähigkeit.

Die Erfindung, wie sie in den Patentansprüchen gekennzeichnet ist, löst demnach die Aufgabe, ein Verfahren und eine Vorrichtung der genannten Art zu schaffen, mit denen Anteile fester Stoffe in einem Prüfgut einfach und sicher auch für durchsichtige Stoffe oder für Stoffe mit ähnlicher Farbe wie das Prüfgut ermittelt werden können.

Dies wird dadurch erreicht, dass das Prüfgut einem elektrischen Feld ausgesetzt und dielektrische Eigenschaften des Feldes mit dem Prüfgut ermittelt werden. Zur Bestimmung der dielektrischen Eigenschaften des Feldes werden elektrische Messgrössen gemessen und daraus mindestens zwei elektrische Grössen ermittelt und kombiniert, wobei ein Kennwert entsteht, der von der Masse des Prüfguts unabhängig ist. Der Kennwert wird mit Vergleichswerten verglichen und aus dem Vergleich erhält man eine Angabe über den Anteil fester Stoffe oder dessen Veränderung im Prüfgut. Die dielektrischen Eigenschaften können anhand mehrerer elektrischer Grössen erfasst werden.

Eine erste Möglichkeit besteht beispielsweise darin, aus Messgrössen wie der Spannung, dem Strom, der Phasenverschiebung zwischen Spannung und Strom sowie allfälligen Vergleichswiderständen, als elektrische Grösse, die durch das Prüfgut bewirkte Kapazitätsänderung oder die relative Dielektrizitätskonstante εᵣ in einem elektrischen Wechselfeld bei mindestens zwei Frequenzen zu bestimmen und daraus einen Quotienten als Kennwert zu bilden.

Eine zweite Möglichkeit besteht beispielsweise darin, aus Messgrössen wie der Spannung, dem Strom, der Phasenverschiebung zwischen Spannung und Strom sowie allfälligen Vergleichswiderständen elektrische Grössen wie den Leistungsfaktor cos Φ der durch das Prüfgut bewirkten Kapazitätsänderung und damit des Prüfgutes selber als Kennwert zu bestimmen.

Der aus den elektrischen Grössen beispielsweise durch Quotientenbildung ermittelte Kennwert bleibt konstant, solange der Anteil fester Stoffe im Prüfgut konstant bleibt. Ändert sich der Anteil, so wird dies durch eine entsprechende Änderung des Kennwertes angezeigt. Aus dem konstanten Kennwert und aus dem geänderten Kennwert, lässt sich auch der absolute Anteil fester Stoffe im Prüfgut durch Verhältnisbildung ermitteln.

Die Vorrichtung besteht aus mindestens einem Messkondensator, der zur Bildung eines elektrischen Feldes im Bereiche des Prüfguts angeordnet ist, einem daran angeschlossenen Frequenzgenerator, zur Erzeugung von mindestens einer Frequenz im elektrischen Feld, daran angeschlossenen Messelementen für elektrische Messgrössen und einer Auswerteschaltung zur Bildung der elektrischen Grössen und des Kennwertes und zum Vergleichen des Kennwertes mit vorgegebenen Werten.

Um die Grundkapazität des Kondensators ohne Prüfgut ausschalten zu können, kann ein Referenzkondensator an der gleichen oder an einer invertierten Signalquelle angeschlossen, vorgesehen sein. Vorzugsweise bilden die Frequenzgeneratoren mit dem Messkondensator und dem Referenzkondensator eine Brückenschaltung.

Die dielektrischen Eigenschaften eines Feldes werden durch mindestens eine Grösse aus einer Gruppe von elektrischen Grössen enthaltend die Kapazität, die Dielektrizitätszahl, den Vertustwinkel und den Leistungsfaktor dargestellt.

Die durch die Erfindung erreichten Vorteile sind insbesondere darin zu sehen, dass damit verschiedenartigste Fremdstoffe, Zusammensetzungen oder Anteile fester Stoffe in einem Prüfgut erfasst werden können. Dies, ob nun gewisse Stoffe sichtbar, unsichtbar oder von ähnlicher Farbe wie das Prüfgut, im Inneren oder an der Oberfläche vorkommen. Die zur Durchführung des Verfahrens vorgesehenen Mittel sind einfach im Aufbau und erlauben es problemlos mit anderen Messvorrichtungen kombiniert zu werden, die andere Ziele verfolgen indem sie andere Parameter messen.

Im folgenden wird die Erfindung anhand eines Beispiels und mit Bezug auf die beiliegenden Figuren näher erläutert. Es zeigen:
Figur 1 ein eine schematische und vereinfachte Darstellung einer ersten Vorrichtung,
Figuren 2 und 3 je eine grafische Darstellung physikalischer Zusammenhänge und
Figuren 4 bis 6 je weitere Ausführungen der erfindungsgemässen Vorrichtung.

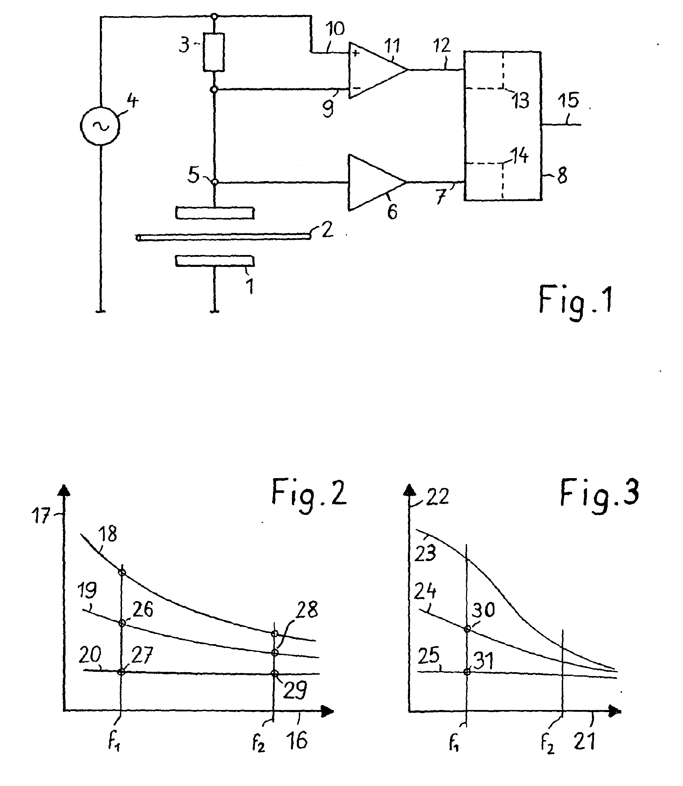

Fig. 1 zeigt eine erste Ausführung einer erfindungsgemässen Vorrichtung mit einem Messkondensator 1 für ein langgestrecktes und längsbewegtes Prüfgut 2 wie beispielsweise ein Garn, Vorgarn, Band, Filament usw. Der Messkondensator 1 ist einerseits über einen Widerstand 3 mit einem Frequenzgenerator 4 und andererseits mit der Erde verbunden. Ein Ausgang 5 des Messkondensators 1 ist über einen Verstärker 6 mit einem Eingang 7 einer Auswerteschaltung 8 verbunden. Derselbe Ausgang 5 ist auch an einen Eingang 9 und über den Widerstand 3 an den anderen Eingang 10 eines Operationsverstärkers 11 angeschlossen, der zum Widerstand 3 parallel geschaltet ist. Der Ausgang des Operationsverstärkers 11 bildet einen weiteren Eingang 12 für die Auswerteschaltung 8. Die Eingänge 7 und 12 sind in der Auswerteschaltung 8 mit an sich bekannten Messelementen 13, 14 für elektrische Grössen verbunden. Die Auswerteschaltung 8 weist einen Ausgang 15 auf.

Fig. 2 zeigt eine grafische Darstellung mit zwei Achsen 16 und 17. Längs der Achse 16 sind Werte für Frequenzen eines elektrischen Feldes und längs der Achse 17 sind Werte für die relative Dielektrizitätszahl εᵣ des gleichen Feldes aufgetragen. Kurven 18, 19 und 20 geben somit die relative Dielektrizitätszahl εᵣ als Funktion der Frequenz für Felder mit einem Prüfgut aus Baumwolle mit 68% Feuchte, aus Baumwolle mit 47% Feuchte und aus Polypropylen an. Man erkennt hier, dass die relative Dielektrizitätszahl für Polypropylen gemäss Kurve 20 weitgehend unabhängig von der Frequenz ist, während die relative Dielektrizitätszahl für Baumwolle eine starke Frequenzabhängigkeit aufweist, wie die Kurven 18 und 19 zeigen. Mit wachsendem Feuchtigkeitsgehalt wächst die Frequenzabhängigkeit. Da die Kapazitätsänderung des Messkondensators proportional mit der Dielektrizitätszahl ändert, gilt für die Kapazitätsänderung die gleiche relative Abhängigkeit von der Frequenz. Gleichzeitig wird aber die Kapazitätsänderung auch von der Masse des Prüfguts beeinflusst.

Fig. 3 zeigt eine grafische Darstellung mit zwei Achsen 21 und 22. Längs der Achse 21 sind Werte für Frequenzen eines elektrischen Feldes und längs der Achse 22 sind Werte für den Leistungsfaktor cosΦ des Prüfguts, für verschiedene Materialien aufgetragen. Kurven 23, 24 und 25 geben somit den Leistungsfaktor cosΦ als Funktion der Frequenz für Baumwolle mit 68% Feuchte, für Baumwolle mit 47% Feuchte und für Polypropylen als Prüfgut an. Während der Leistungsfaktor von Polypropylen weitgehend unabhängig von der Frequenz ist, weist der Leistungsfaktor von Baumwolle eine starke Abhängigkeit von der Frequenz auf. Auch hier steigt die Abhängigkeit mit wachsendem Feuchtigkeitsgehalt der Baumwolle. Dagegen ist der Leistungsfaktor der Kapazitätsänderung des Messkondensators unabhängig von der Masse des Prüfguts.

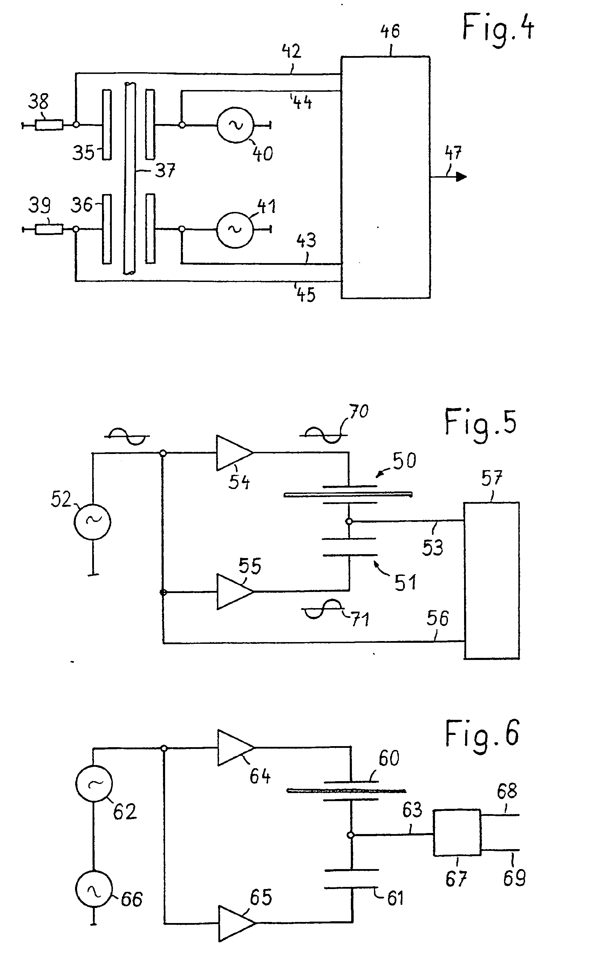

Fig. 4 zeigt eine zweite Ausführung einer erfindungsgemässen Vorrichtung mit zwei Messkondensatoren 35 und 36 für ein langgestrecktes und längsbewegtes Prüfgut 37 wie beispielsweise ein Garn, Vorgarn, Band, Filament usw. Die Messkondensatoren 35, 36 sind einerseits über Widerstände 38, 39 mit der Erde und andererseits mit je einem Frequenzgenerator 40, 41 verbunden. Die Messkondensatoren 35, 36 sind über Leitungen 42, 44 und 43, 45 mit einer Auswerteeinheit 46 verbunden, die beispielsweise als Prozessor ausgebildet ist und einen Ausgang 47 aufweist.

Fig. 5 zeigt eine dritte Ausführung in vereinfachter Darstellung mit einem Messkondensator 50, einem Referenzkondensator 51 und einem Frequenzgenerator 52. Ein Abgriff 53 liegt zwischen den Kondensatoren 50 und 51 und beiden ist je ein invertierender- oder nicht-invertierender Verstärker 54, 55 zugeordnet. Ferner ist eine Auswerteschaltung 57 vorgesehen, die über den Abgriff 53 und eine Leitung 56 angeschlossen ist.

Fig. 6 zeigt eine vierte Ausführung in vereinfachter Darstellung mit einem Messkondensator 60, einem Referenzkondensator 61 und einem Frequenzgenerator 62. Ein Abgriff 63 liegt zwischen den Kondensatoren 60 und 61, und beiden ist je ein invertierender- oder nicht-invertierender Verstärker 64, 65 zugeordnet. Hier ist auch ein weiterer Frequenzgenerator 66 zum Frequenzgenerator 62 in Serie geschaltet. Der Abgriff 63 mündet in ein Element zur Frequenztrennung 67 mit zwei Ausgängen 68, 69.

Die Wirkungsweise der Erfindung ist wie folgt:
Um Anteile fester Stoffe wie beispielsweise den Anteil von Polypropylen in einem Baumwollgarn oder Baumwollband zu bestimmen, wird das Prüfgut und damit das Baumwollgarn oder Baumwollband in ein elektrisches Wechselfeld gelegt, das mit Wechselstrom einer bestimmten Frequenz f1 erzeugt wird. Damit entsteht ein elektrisches Wechselfeld dessen dielektrische Eigenschaften durch messbare Werte wie Strom, Spannung und Phasenwinkel, aber auch durch die vorgegebene Frequenz bestimmt sind. Die dielektrischen Eigenschaffen können dabei insbesondere durch die relative Dielektrizitätszahl εᵣ und/oder den Leistungsfaktor cosΦ ausgedrückt werden. Man kann diesen Vorgang auch in weiteren elektrischen Wechselfeldern mit weiteren Frequenzen f2, f3 usw. wiederholen, und erhält dabei auch zweite, dritte usw. abweichende Werte für die relative Dielektrizitätszahl εᵣ und den Leistungsfaktor cosΦ.

Kennt man die Werte, die die dielektrischen Eigenschaften ausdrücken, für die Stoffe, die im Prüfgut vorhanden sind getrennt, was beispielsweise in den Figuren 2 und 3 dargelegt ist, so lässt sich daraus der Anteil der Stoffe im Prüfgut bestimmen. Dazu gibt es verschiedene Möglichkeiten.

Eine erste Möglichkeit besteht darin, aus den dielektrischen Eigenschaften und der Stoffmenge, die Änderung der Kapazität Δ C des Messkondensators als Folge eines darin eingeführten Prüfguts zu bestimmen und zwar bei mindestens zwei verschiedenen Frequenzen f1, f2,... des Messfeldes im Messkondensator. Dies einerseits für reines Prüfgut wie Polypropylen oder Baumwolle rechnerisch mit Werten aus der Fig. 2. und andererseits durch Messung entsprechender Werte der dielektrischen Eigenschaften am Messkondensator mit realem Prüfgut. Geht man davon aus, dass das Prüfgut aus zwei Stoffen besteht, so kann mit Werten aus der Figur 2 die genannte Kapazitätsänderung des Messkondensators mit einem Prüfgut aus je einem der Stoffe allein berechnet werden. Daraus kann die Kapazitätsänderung des Messkondensators bei reinem und bei realem Prüfgut bei zwei oder mehreren Frequenzen ermittelt werden. Aus den ermittelten Kapazitätsänderungen bei verschiedenen Frequenzen für das Prüfgut, kann als Kennwert ein Quotient ermittelt werden, der beispielsweise für Polypropylen idealerweise = 1, für Baumwolle > oder < 1 ist. Dieser Kennwert ist nun von der Masse des Prüfguts unabhängig und kann für reales Prüfgut laufend überwacht werden, indem sie mit einem Referenzwert verglichen wird. Tritt eine Abweichung auf, so hat sich der Anteil des Fremdstoffes im Prüfgut verändert. Oder, aus einer nachfolgend wiedergebenden Formel kann mit dem Kennwert für das reine Prüfgut und dem Kennwert für das reale Prüfgut der Anteil des reinen Stoffes im realen Prüfgut quantifiziert werden.

Eine zweite Möglichkeit besteht darin, aus den elektrischen Messgrössen, die ein elektrisches Feld charakterisieren, den Leistungsfaktor cosΦ der durch das, in das Feld eingeführte, Prüfgut bewirkten, Kapazitätsänderung in bekannter Weise zu bestimmen. Dieser Leistungsfaktor ist aber von der Masse des Prüfguts unabhängig, weshalb es genügt den Leistungsfaktor für reines Prüfgut und den Leistungsfaktor für reales Prüfgut zu bestimmen. Aus den beiden Leistungsfaktoren kann gemäss einer nachfolgend wiedergegebenen Formel ein Kennwert bestimmt werden, der proportional zum Anteil eines der Stoffe im Prüfgut ist und die ebenfalls überwacht werden kann.

In der Vorrichtung gemäss Fig. 1 wird das Prüfgut 2 im Messkondensator 1 einem elektrischen Feld mit einer Frequenz f1 ausgesetzt. Der Messkondensator 1 wird vom Frequenzgenerator 4 über den Widerstand 3 mit entsprechenden Wechselströmen gespeist, so dass sich im Spalt des Messkondensators 1 ein Wechsel-Feld mit einer Frequenz f1 ergibt. Die Spannung, die am Messkondensator 1 am Ausgang 5 auftritt, wird im Verstärker 6 verstärkt und am Eingang 7 angelegt, so dass diese Spannung im Messelement 14 quantifiziert und anschliessend der Auswerteschaltung 8 zugeführt wird. Die über dem Widerstand 3 auftretende Spannung wird im Operationsverstärker 11 verstärkt und über den Eingang 12 dem Messelement 13 zugeführt wo diese ebenfalls quantifiziert wird. Da der Widerstand 3 bekannt ist, kann die Auswerteschaltung 8 daraus auch den Strom im Widerstand 3 berechnen. Daraus und aus den für den Messkondensator 1 bekannten festen Grössen lässt sich die Kapazitätsänderung des Messkondensators 1, die durch das Prüfgut bewirkt wird, nach an sich bekannten Gesetzen ermitteln. Durch Messung der Phasendifferenz zwischen Strom und Spannung wird zudem der Leistungsfaktor cosΦ bestimmt. Die Werte für den Leistungsfaktor cosΦ der Kapazitätsänderung sind von der Masse des Prüfguts 2 im Messkondensator 1 unabhängig. Diese Kapazitätsänderung wird in der Auswerteschaltung 8 mit Werten aus den Messelementen 13, 14 und weiteren festen Eingaben ermittelt. Die Auswerteschaltung 8 kann als elektrische Schaltung oder als Rechner ausgebildet sein, der Messwerte und Eingaben digital verarbeitet. Über den Ausgang 15 liefert die Auswerteschaltung ein Signal, das das Vorhandensein von weiteren festen Stoffen im Prüfgut oder einen veränderten Anteil eines der Stoffe anzeigt. Ein solches Signal kann von der Auswerteeinheit 8 laufend ausgegeben, darin zu Mittelwerten verarbeitet und mit solchen Mittelwerten oder anderen Referenzwerten verglichen werden. Markante Abweichungen deuten dann auf Änderungen der Zusammensetzung des Prüfguts 2 hin.

Für die Quantifizierung des Anteils eines festen Stoffes im Prüfgut 2 geht man davon aus, dass die Stoffe, die das Prüfgut 2 bilden, bekannt sind. Beispielsweise soll das Prüfgut 1 aus Baumwolle mit 47% Feuchte und eventuell Polypropylen bestehen. Aus der Fig. 3 sind Leistungsfaktoren für diese Stoffe bei beliebigen Frequenzen f1, f2 ablesbar. Unter der Annahme, dass die Anteile beider Stoffe zusammen 100% der Masse des Prüfguts 2 ergeben, kann man den Anteil des ersten Stoffes gemäss den nachstehenden Formeln (1) und (2) ermitteln.

In der Vorrichtung gemäss Fig. 4 wird das Prüfgut nacheinander durch zwei Messkondensatoren 35, 36 hindurch bewegt, die Felder verschiedener Frequenzen f1, f2, erzeugen, wie dies durch Wechselspannungen erzeugt ist, die die Frequenzgeneratoren 40, 41 liefern. Die Auswerteeinheit 46 erhält über die Leitungen 42 bis 45 somit Werte für Ströme und Spannungen bei zwei verschiedenen Frequenzen parallel. Daraus lassen sich Kapazitätsänderungen und daraus wiederum ein Kennwert berechnen. Dazu sind in der Auswerteeinheit 46 alle Werte, wie sie in der Figur 2 dargestellt sind, als Tabellen gespeichert. Somit ergibt sich der Anteil eines ersten Stoffes K1 im Prüfgut aus der

Formel (1): K1 = (A - F2)/(F1 - F2).

Der Anteil des zweiten Stoffes ergibt sich aus der

Formel (2): K2 = (A - F1)/(F2 - F1).

Dabei sind die Werte F1 und F2 die Verhältnisse der relativen Dielektrizitätszahlen von Baumwolle und Polypropylen bei der ersten Frequenz f1 und bei der zweiten Frequenz f2. Die Faktoren F1 und F2 ergeben sich deshalb durch Quotientenbildung beispielsweise der Werte 26/28 und 27/29 aus Fig. 2. A entspricht dem Quotienten der Kapazitätsänderungen im Messkondensator für das reale Prüfgut oder dem bereits genannten Kennwert.

In der Vorrichtung gemäss Fig. 5 wird im Frequenzgenerator 52 eine Wechselspannung mit einer Frequenz erzeugt, die den beiden Verstärkern 54, 55 zugeführt und von diesen an beide Kondensatoren 50, 51 angelegt wird. Beispielsweise im Verstärker 55 wird eine Phasenverschiebung um 180° durch eine Verzögerung des Signals erzeugt, so dass die Kondensatoren 50, 51 je mit einem Signal 70, 71 beaufschlagt werden, die einander aufheben. So liegt am Abgriff 53 mindestens dann die Spannung Null, wenn kein Prüfgut im Messkondensator 50 vorhanden ist. So werden alle Einflüsse der leeren Kondensatoren neutralisiert. Das Signal im Abgriff 53 verändert sich sobald ein Prüfgut im Messkondensator 50 eingeführt wird. Der cos des Phasenwinkels des Signals ist invers zum Leistungsfaktor cosΦ des Prüfguts im Messkondensator 50 und unabhängig von der Menge des Prüfguts. Aus dem Leistungsfaktor lässt sich durch eine einfache Mischrechnung mit den Werten aus Fig. 3 für die Frequenz der angelegten Wechselspannung das Mischungsverhältnis der beiden Stoffe im Prüfgut ermitteln. Dies geschieht in der Auswerteschaltung 57, die über die Leitung 56 auch eine Angabe über den Phasenwinkel des unverstärkten und durch die Kondensatoren 50 und 51 unbeeinflussten Signales des Frequenzgenerators 52 erhält.

In der Vorrichtung gemäss Fig. 6 sind die Vorgänge identisch zu jenen in der Vorrichtung gemäss Fig. 5, allerdings mit dem Unterschied, dass von den beiden Frequenzgeneratoren 62, 66 ein Signal mit zwei überlagerten Frequenzen an den Kondensatoren 60, 61 anliegt, das im Element 67 in seine beiden Frequenzen zerlegt wird. Wie für die Vorrichtung gemäss Fig. 4 bekannt, kann das Verhältnis der beiden Komponenten im Prüfgut ermittelt werden. Diese Ausführung, wie auch die Ausführung gemäss Fig. 5, hat gegenüber der Ausführung gemäss Fig. 4 den Vorteil, dass durch das Prinzip der Brückenschaltung der Einfluss des leeren Kondensators kompensiert wird. Beim leeren Messkondensator ist die Signalspannung Null und diese ändert proportional mit den dielektrischen Eigenschaften des Prüfguts und dessen Menge.

Da sich mit diesen Verfahren die Anteile fester Stoffe in einem Prüfgut ermitteln lassen, ist es eine Voraussetzung, dass Anteile nicht fester Stoffe wie Wasser in einem der Stoffe für die Messung konstant sind. Beispielsweise muss die Feuchte in der Baumwolle vor der Messung bekannt und konstant sein. Doch dies ist in der Textilindustrie, ohnehin der Fall, denn die meisten Vorgänge bei der Verarbeitung der Rohstoffe finden in klimatisierten Räumen statt.

Die kapazitive Erkennung fremder Stoffe und deren Anteile in einem Prüfgut kann mit der an sich bekannten Messung anderer Parameter wie z.B. der Gleichmässigkeit, der Masse usw. kombiniert werden, da ein Signal sowohl für die Messung solcher Parameter wie auch für die Erkennung der Anteile fremder Stoffe benützt werden kann. Die Anteile fremder Stoffe können wie erwähnt durch Berechnung der Kapazität bei mehreren Frequenzen oder durch Berechnung des Leistungsfaktors bei nur einer Frequenz ermittelt werden. Es ist aber auch denkbar, dies über die Berechnung des Leistungsfaktors für mehrere Frequenzen zu tun. Geeignete Frequenzen betragen beispielsweise 10 kHz bis 100 kHz und 10 MHz.

## Patentansprüche

1. Verfahren zur Ermittlung von Anteilen fester Stoffe in einem langgestreckten und längsbewegten Prüfgut (2), **dadurch gekennzeichnet, dass**
in einem Messkondensator (1) ein elektrisches wechselfeld gebildet wird,
das Prüfgut in das elektrische Feld gelegt wird,
im Feld elektrische Grössen gemessen und daraus dielektrische Eigenschaften des elektrischen Feldes bestimmt werden,
durch Kombination zweier elektrischer Grössen des elektrischen Feldes ein Kennwert gebildet wird, der von der Masse des Prüfguts unabhängig ist und
durch Vergleich des Kennwertes mit Vergleichswerten eine Angabe über den Anteil fester Stoffe im Prüfgut erhalten wird.

2. Verfahren nach Anspruch 1, **dadurch gekennzeichnet, dass** das elektrische Feld mit einer ersten Frequenz und einer zweiten Frequenz betrieben wird und der Anteil fester Stoffe aus Werten für dielektrische Eigenschaften ermittelt wird, die bei der ersten und zweiten Frequenz gemessen werden.

3. Verfahren nach Anspruch 1, **dadurch gekennzeichnet, dass** im elektrischen Feld bei einer Frequenz, als Kennwert der elektrische Leistungsfaktor bestimmt wird.

4. Verfahren nach Anspruch 1, **dadurch gekennzeichnet, dass** das Prüfgut ein Garn, ein Vorgarn, Band oder ein Filament ist.

5. Verfahren nach Anspruch 2, **dadurch gekennzeichnet, dass** aus dem Feld mit der ersten Frequenz und aus dem Feld mit der zweiten Frequenz je ein Messwert gewonnen wird und die beiden Messwerte zu einem Signal verknüpft werden, das mit einem vorgegebenen Wert verglichen wird.

6. Verfahren nach Anspruch 5, **dadurch gekennzeichnet, dass** als Messwerte Ströme, Spannungen und Phasenwinkel gemessen und daraus dielektrische Eigenschaften bestimmt werden.

7. Vorrichtung zur Durchführung des Verfahrens nach Anspruch 1,
**gekennzeichnet durch**
einen Messkondensator (1) zur Bildung eines elektrischen wechselfeldes im Bereiche des Prüfguts (2),
einen Frequenzgenerator (4) zur Erzeugung von mindestens einem elektrischen Feld mit einer Frequenz,
daran angeschlossene Messelemente (13, 14) für elektrische Messgrössen und
eine mit den Messelementen (13,14) verbundene Auswerteschaltung (8), die zur Bildung eines von der Masse des Prüfguts unabhängigen Kennwertes durch Kombination zweier elektrischer Grössen des elektrischen Feldes und
zur Angabe über den Anteil fester Stoffe im Prüfgut **durch** Vergleich des Kennwertes mit Vergleichswerten
eingerichtet ist.

8. Vorrichtung nach Anspruch 7, **dadurch gekennzeichnet, dass** neben dem Messkondensator (50) ein Referenzkondensator (51) vorgesehen ist.

9. Vorrichtung nach Anspruch 7, **dadurch gekennzeichnet, dass** zwei Frequenzgeneratoren (62, 66) vorgesehen sind.

10. Vorrichtung nach Anspruch 7, **dadurch gekennzeichnet, dass** die Frequenzgeneratoren in Serie geschaltet sind, zur Erzeugung eines Elektrischen Feldes mit zwei einander überlagerten Frequenzen.

11. Vorrichtung nach Anspruch 8, **dadurch gekennzeichnet, dass** der Messkondensator (50) und der Referenzkondensator (51) Teil einer Brückenschaltung sind.

## Claims

1. A method for determining the proportions of solid materials in a longitudinally extended and longitudinally moved sample (2), **characterized in that**
an electric alternating field is formed in a measuring capacitor (1),
the sample is placed in the electric field,
electric values are measured in the field and the dielectric properties of the electric field are determined therefrom,
a characteristic value is formed by combination of two electric values of the electric field, which characteristic value is independent of the mass of the sample and
information on the proportion of solid materials in the sample is obtained by comparison of the characteristic value with comparative values.

2. The method according to claim 1, **characterized in that** the electric field is operated with a first frequency and a second frequency and the proportion of solid materials is determined from values for dielectric properties which are measured in the first and second frequency.

3. The method according to claim 1, **characterized in that** the electric power factor is determined as the characteristic value at a frequency in the electric field.

4. The method according to claim 1, **characterized in that** the sample is a yarn, a roving, a sliver or a filament.

5. The method according to claim 2, **characterized in that** one measured value each is obtained from the field with the first frequency and the field with the second frequency and the two measured values are combined into a signal which is compared with a predetermined value.

6. The method according to claim 5, **characterized in that** currents, voltages or phase angles are measured as measured values and dielectric properties are determined therefrom.

7. An apparatus for performing the method according to claim 1,
**characterized by**
a measuring capacitor (1) for forming an electric alternating field in the region of the sample (2),
a frequency generator (4) for generating at least one electric field with a frequency,
and measuring elements (13, 14) connected thereto for electric measured values, and
an evaluation circuit (8) which is connected with the measuring elements (13, 14) and which is set up
for forming a characteristic value which is independent of the mass of the sample by combination of two electric values of the electric field and
for stating the proportion of solid materials in the sample by comparing the characteristic value with comparative values.

8. The apparatus according to claim 7, **characterized in that** a reference capacitor (51) is provided in addition to the measuring capacitor (50).

9. The apparatus according to claim 7, **characterized in that** two frequency generators (62, 66) are provided.

10. The apparatus according to claim 7, **characterized in that** the frequency generators are connected in series, for generating an electric field with two frequencies superimposed on one another.

11. The apparatus according to claim 8, **characterized in that** the measuring capacitor (50) and the reference capacitor (51) are part of a bridge circuit.

## Revendications

1. Procédé de détermination des fractions de corps solides dans un échantillon allongé et mobile en longueur (2), **caractérisé en ce**ci que,
dans un condensateur de mesure (1), un champ électrique alternatif est formé, l'échantillon à tester est posé dans ledit champ électrique,
dans ledit champ électrique, des grandeurs électriques sont mesurées et ainsi les propriétés diélectriques dudit champ sont déterminées,
par combinaison de deux valeurs de mesure dudit champ électrique un indice est formé, ledit indice étant indépendant de la masse dudit échantillon à tester et
par comparaison de l'indice à des valeurs de comparaison une indication sur ladite fraction de corps solides est obtenue.

2. Procédé selon la revendication 1, **caractérisé en ceci que** ledit champ électrique est utilisé avec une première fréquence et avec une seconde fréquence et que ladite fraction de corps solides est déterminée à partir des valeurs des propriétés diélectriques mesurées pour la première et pour la seconde fréquence.

3. Procédé selon la revendication 1, **caractérisé en ce**ci que, dans ledit champ électrique pour l'une des fréquences, le facteur de puissance est déterminé comme indice.

4. Procédé selon la revendication 1, **caractérisé en ce**ci que ledit échantillon à tester est un fil, une mèche, un ruban, ou un filament.

5. Procédé selon la revendication 2, **caractérisé en ce**ci qu'une valeur de mesure est acquise à partir du champ ayant la première fréquence et une à partir du champ ayant la seconde fréquence et que les deux valeurs de mesure sont combinées pour constituer un signal, ledit signal étant comparé à une valeur prédéterminée.

6. Procédé selon la revendication 5, **caractérisé en ce**ci que comme valeurs de mesure des courants, des tensions et des angles de phase sont mesurés et à partir d'eux des propriétés diélectriques sont déterminées.

7. Dispositif pour la réalisation du procédé selon la revendication 1,
**caractérisé par**
un condensateur de mesure (1) pour la formation d'un champ électrique alternatif au niveau dudit échantillon à tester (2),
un générateur de fréquence (4) pour générer au moins un champ électrique ayant une fréquence,
d'éléments de mesure (13, 14) branchés sur ledit générateur pour des grandeurs de mesure électriques et
un circuit de restitution (8) relié aux éléments de mesure (13, 14), lequel est disposé pour former un indice indépendant de la masse dudit échantillon à tester par combinaison de deux grandeurs électriques dudit champ électrique et pour indiquer ladite fraction de corps solides dans un échantillon à tester en comparant l'indice à des valeurs de comparaison.

8. Dispositif selon la revendication 7, **caractérisé en ce**ci qu'en plus dudit condensateur de mesure (50) un condensateur de référence (51) est prévu.

9. Dispositif selon la revendication 7, **caractérisé en ce**ci que deux générateurs de fréquence (62, 66) sont prévus.

10. Dispositif selon la revendication 7, **caractérisé en ce**ci que lesdits générateurs de fréquence sont branchés en série pour générer un champ électrique ayant deux fréquences interférant l'une avec l'autre.

11. Dispositif selon la revendication 8, **caractérisé en ce**ci que ledit condensateur de mesure (50) et ledit condensateur de référence (51) font partie d'un circuit en pont.
